# EUROPEAN PATENT SPECIFICATION

(11) **EP 1 414 524 B1**
(45) Date of publication and mention of the grant of the patent: **22.03.2006**
(21) Application number: 02754899.9
(22) Date of filing: 17.07.2002
(51) Int. Cl.: A61P 17/14, A61K 38/03, A61K 38/04

(54) **TREATMENT OF HAIR FOLLICLE WITH NEUROKININ 1 RECEPTOR ANTAGONISTS**
BEHANDLUNG VON HAARFOLLIKELN MIT NEUROKININ 1 REZEPTOR ANTAGONISTEN
TRAITEMENT DU FOLLICULE PILEUX A L'AIDE D'ANTAGONISTES DU RECEPTEUR DE LA NEUROKININE 1

(30) Priority: 23.07.2001 IT PD20010187
(43) Date of publication of application: 06.05.2004
(73) Proprietor: Cutech S.r.l., 35127 Padova (IT)
(72) Inventor: PAUS, Ralf, 22453 Hamburg (DE); ARCK, Petra, Clara, 14482 Potsdam (DE)
(74) Representative: Fabry, Bernd
(86) International application number: PCT/EP2002/007963
(87) International publication number: WO 2003/009898

(56) References cited:
- US-A- 5 958 432
- US-A- 5 972 938
- PETERS E M ET AL: "Hair growth-modulation by adrenergic drugs." EXPERIMENTAL DERMATOLOGY. DENMARK AUG 1999, vol. 8, no. 4, August 1999 (1999-08), pages 274-281, XP001118198 ISSN: 0906-6705
- BOTCHKAREV V A ET AL: "Hair cycle-dependent changes in adrenergic skin innervation, and hair growth modulation by adrenergic drugs." THE JOURNAL OF INVESTIGATIVE DERMATOLOGY. UNITED STATES DEC 1999, vol. 113, no. 6, December 1999 (1999-12), pages 878-887, XP001118141 ISSN: 0022-202X
- SPÄTLING L ET AL: "[Increased hair growth during prolonged tocolytic therapy with fenoterol. Measurements of testosterone, androstandiol, cortisol and ACTH (author's transl)]" GEBURTSHILFE UND FRAUENHEILKUNDE. GERMANY, WEST NOV 1980, vol. 40, no. 11, November 1980 (1980-11), pages 1022-1028, XP001107062 ISSN: 0016-5751
- ARCK P C ET AL: "Indications for a 'brain-hair follicle axis (BHA)': inhibition of keratinocyte proliferation and up-regulation of keratinocyte apoptosis in telogen hair follicles by stress and substance P." THE FASEB JOURNAL: OFFICIAL PUBLICATION OF THE FEDERATION OF AMERICAN SOCIETIES FOR EXPERIMENTAL BIOLOGY; EXPRESS ARTICLE 10.1096/FJ.00-0699FJE, [Online] 17 September 2001 (2001-09-17), pages 1-26, XP002217394 UNITED STATES Retrieved from the Internet: <URL:http://www.fasebj.org/cgi/doi/10.1096 /fj.00-0699fje> [retrieved on 2002-10-18]
- PAUS R ET AL: "Hair growth induction by substance P." LABORATORY INVESTIGATION;A JOURNAL OF TECHNICAL METHODS AND PATHOLOGY. UNITED STATES JUL 1994, vol. 71, no. 1, July 1994 (1994-07), pages 134-140, XP001107059 ISSN: 0023-6837

## Description

### Technical field

The present invention relates to the use of a substance for producing a medicament for the treatment of hair follicles, in particular against hair loss.

### Background art

As is known, the life of a hair follicle is characterized by continual and cyclical transition between a growth stage of the follicle (anagen) in which, amongst other things, the development of the hair is observed (by virtue of the activity of the keratinocytes), a subsequent regression stage (catagen) in which the programmed death (apoptosis) of a considerable portion of the cells of the follicle takes place, and a third, quiescence stage (telogen) at the end of which the hair follicle returns to the anagenic stage with the formation of a new hair shaft.

The duration of the various stages of the life cycle of the hair follicle depends substantially on its position on the body. For example, whereas in the scalp region, anagen lasts from two to eight years, compared with a period of a few weeks for the catagen stage and a few months for the telogen stage, in the eyebrow region, the anagen stage lasts for only a few months. This time ratio also determines the percentage of hair follicles which are present, on average, in the various stages of the cycle, for each region of the body.

The durations of the various stages of the cycle, as well as the transition between one stage and another are regulated by complex biological interactions, the mechanisms of which are not entirely clear, between the various parts of the hair follicle and between the follicle and the surrounding epithelial environment. It is, however, known that these stages are affected by many endogenous and exogenous factors which act, directly or indirectly, on the hair follicle to lengthen or shorten the duration of each individual stage.

One of the factors which has been suspected for some time of causing premature catagen of the hair follicle which, in the scalp region, may manifest itself in the form of alopecia or effluvium, giving rise to hair loss, is stress [Paus, R., Peters, E.M., Eichmüller, S., Botchkarev, V.A. (1997): "Neural mechanisms of hair growth control" J. Invest. Dermatol. Symp. Proc. 2, 61-68; Paus, R. "Stress, hair growth control, and the neuro-endocrine-immune connection" J. Allerg. (2001). Ericson M., Gabrielson, A., Worel, S., Lee, W.S., Hordinsky, M.K. (1999) "Substance P (SP) in innervated and non-innervated blood vessels in the skin of patients with symptomatic scalp" Exp. Dermatol. 8, 344-345; Whitlock, F.A. (1976) "Psychophysiological aspects of skin disease" in Rook A. (ed.) *Major Problems in Dermatology,* Vol. 8; Saunders, London].

Stress which, in the sense used in the present context, is intended to include, in addition to conditions resulting from anxiogenic events, also conditions resulting from chemotherapy or pharmacological treatments, causes a systemic reaction characterized by changes in the blood levels of certain transmitters and hormones.

In this connection, some studies have suggested a potentially negative influence of the high level of activity of perifollicular immunocytes (particularly mast cells and macrophages), resulting from the immune response to the stress situation, on the anagen stage of the hair follicle (Maurer, M., Fischer, E., Handjiski, B., von Stebut, E., Algermissen, B., Bavandi, A., Paus, R. (1997) "Activated skin mast cells are involved in murine hair follicle regression (catagen)" *Lab. Invest.* 77, 319-332; Eichmüller, S., van der Veen, C., Moll, I., Hermes, B., Hofmann, U., Müller-Röver, S., Paus, R. (1998) "Clusters of perifollicular macrophages in normal murine skin: physiological degeneration of selected hair follicles by programmed organ deletion" *J. Histochem. Cytochem.* 46, 361-370; Suzuki, S., Kato, T., Takimoto, H., Masui, S., Oshima, H., Ozava, K., Suzuki, S., Imamura, T. (1998) "Localization of rat FGF-5 protein in skin macrophage-like cells and FGF-5S protein in hair follicle: possible involvement of two Ffg-5 gene products in hair growth cycle regulation" *J. Invest. Dermatol.* 111, 963-972; Stenn, K.S., Paus, R. "Control of hair cycle" *Physiol. Rev. Vol. 81, N. 1, (2001)]*

Other studies which, however, relate to other fields of research, have shown that the immune response is mediated particularly but not exclusively by the neuropeptide Substance P (SP), a ligand of the NK1 receptor (Zhu, G.F., Chancellor-Freeland, C., Berman, A.S., Kage, R., Leeman, S.E., Beller, D.I., Black P.H. (1996) "Endogenous substance P mediates cold water stress-induced increase in interleukin-6 secretion from peritoneal macrophages" *J. Neurosci.* 16, 3745-3752; De Felipe, C., Herrero, J.F., O'Brien, J.A., Palmer, J.A., Doyle, C.A., Smith, A.J., Laird, J.M., Belmonte, C., Cervero, F., Hunt, S.P. (1998) "Altered nociception, analgesia and aggression in mice lacking the receptor for substance P" *Nature* 392, 394-397; Nio, D.A., Moylan, R.N., Roche, J.K. (1993) "Modulation of T lymphocyte function by neuropeptides. Evidence for their role as local immunoregulatory elements." *J. immunol.* 150, 5281-5288].

It is also known that the biological action of substance P can be blocked, or at least limited, by the administration of NK1-receptor antagonist substances.

NK1-receptor antagonist substances are currently used in the production of medicaments for the treatment of pathological conditions of the central nervous system (in particular as antidepressants), and respiratory, allergic, cardiovascular, ophthalmic, dermatological and rheumatic pathological conditions in which substance P is thought to be directly involved.

A second use of NK1-receptor antagonist substances as angiogenesis-inhibiting agents, for a hair-growth inhibition treatment, is indicated in United States patent No. 6093748.

Interference of substance P with the normal cyclical behaviour of the hair follicle has also been observed [Paus, R., Heinzelmann, T., Schultz, K.D., Furkert, J., Fechner, K., Czametzki, B.M. (1994) "Hair growth induction by substance P" *Lab. Invest* 71, 134-140]. In fact, when subjected to treatment with substance P during the telogen stage, the hair follicle is induced to go on to the subsequent anagen stage.

However, it is not possible to infer from these results any positive teaching for the preventive treatment of hair follicles which are subject to premature catagen.

### Disclosure of the invention

The main aim of the present invention is to provide a medicament for the treatment of hair follicles, in particular, against hair loss.

Within the scope of this main aim, a first object of the invention is to provide a medicament for the preventive treatment of these pathological conditions.

A second object of the invention is to provide a medicament for the simultaneous anti-inflammatory treatment of the perifollicular region.

These and other objects which will become clearer from the following description are achieved by the present invention by the use of an NK1-receptor antagonist substance in the production of a medicament for the above-mentioned treatment.

The present invention arises from a series of observations which have been drawn from tests in vivo, described in greater detail below, and on the basis of which the following points can be supported scientifically:
- stress induces premature catagen of the hair follicle and leads to an increase in the activity of some immunocytes,
- these stress-induced effects can be simulated by treatment of the hair follicle, in the anagen stage, with substance P,
- these effects can be substantially abrogated by treatment of the hair follicle, in the anagen stage, with an NK1-receptor antagonistic substance.

The tests were carried out in vivo on mice of the strain CBA/J which were pregnant since, as is known, there is a close correlation between exposure to stress and an increase in the percentage of abortions.

In order to determine the effect of stress on hair growth, the following biological and immunological parameters were measured:
- the number of cells in apoptosis in the hair follicle,
- the proliferation of keratinocytes in the hair follicle,
- the activity of some immunocytes, in particular, of macrophages, mast cells, and γδ T cells.

In accordance with a well-established model for the study of stress, some mice were subjected to predetermined doses of ultrasound (sonic stress) for 7 days, to ascertain its impact both on the activity of the hair follicle and on the immune response of the perifollicular region.

Another group of mice was subjected to treatment with substance P (SP), and yet others to sonic stress treatment combined with the administration of a NK1-receptor antagonist substance (indicated SP-RA).

Upon completion of the above-mentioned treatments, the parameters listed above were measured again and compared with those measured in mice which had not been subjected to any treatment.

The results of the tests described above are summarized in Table 1 below.

**Table 1**

| **Treatment of the mice** | **% Abortions** | **TUNEL**^{**+**} | **TUNEL**^{**+**} | **Ki67**^{**+**} | **MHC II** | **MC** | **γδ TCR**^{**+**} |
|---|---|---|---|---|---|---|---|
| **No stress** | 12 | 2.2 | 3.6 | 8.6 | 5.4 | 3.5 | 1.9 |
| **Induced Stress** | 39 | 10.2 | 18.0 | 6.4 | 10.4 | 4.9 | 0.7 |
| **No stress + SP** | 19 | 8.0 | 9.0 | 7.7 | 11.0 | 7.9 | 1.3 |
| **Stress + SP-RA** | 24 | 1.6 | 3.3 | 5.9 | 3.3 | 5.0 | 1.5 |

The number of cells in apoptosis was measured by the TUNEL technique in a first region of the hair follicle comprising the infundibulum, the capsular germinal region, and the secondary germinal region (column 3), and in a second, more extensive region comprising, in addition to the first region, the epidermis and the parafollicular dermis (column 4).

The proliferation of the keratinocytes was measured by an immunohistochemical technique by measurement of the positive Ki67 cells (column 5).

Macrophage presence was investigated by detection of the expression of Class II MHC glycoprotein (column 6).

Mast-cell activity was measured by measuring the percentage of degranulated mast cells relative to the total mast cells present in the field of vision (column 7).

γδ T-cell presence was detected by an immunohistochemical technique (column 8).

With reference to the data given in the first and second lines of Table 1, it can easily be seen that the stress situation induced in the mice leads to a considerable increase in the cells in apoptosis and to a marked reduction in the proliferative activity of the keratinocytes, confirming experimentally that stress causes premature catagen of the hair follicle.

The stress situation also leads to a marked immune response with an appreciable increase in the perifollicular macrophage groups and in the percentage of degranulated mast cells, and a simultaneous reduction in γδ T cells. This response also leads to a state of inflammation of the perifollicular region.

The third line of data of Table 1 shows that the administration of substance P simulates, to a large extent, the biological and immune effects of stress. In fact, a considerable increase in the cells in apoptosis, a reduction in the keratinocyte activity, an increase in macrophages and mast cells, and a reduction in γδ T cells are noted.

Finally, it can be seen from an examination of the fourth line of Table 1 that the administration of an NK1-receptor antagonist substance limits and, for some aspects, cancels out, the effect of the stress induced. In particular, the level of cells in apoptosis has returned to normal and the immune activity attributable to the macrophages and to the γδ T cells is considerably reduced.

To confirm the results obtained, further tests were carried out on groups of mice of the same type as those used previously, in which the extent of progress of the cycle (HCS) was measured in accordance with the method suggested by Maurer et al. in American Journal of Pathology of 1997, 16 days after epilation treatment. The extent of progress of the cycle in this case was an index of the rate at which the hair follicle progresses from the anagen to the catagen.

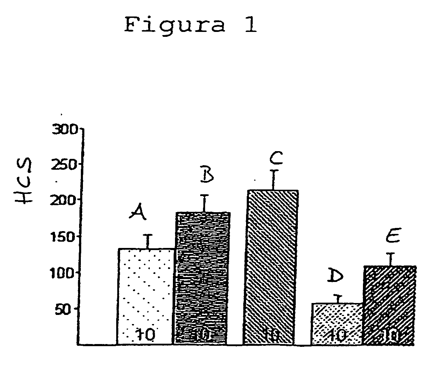

With reference to the histogram of Figure 1, appended to the present description:
- the first group of mice (A) underwent no treatment,
- the second group (B) was subjected to sonic stress,
- the third group (C) was subjected to treatment with substance P,
- the fourth group (D) was subjected to treatment with an NK1-receptor antagonist substance, and
- the fifth group (E) was subjected to combined stress and NK1-receptor antagonist substance treatment.

As is clear from the results shown in the histogram of Figure 1, stress causes premature catagen which is an effect also well simulated by substance P, and treatment with an NK1-receptor antagonist substance advantageously leads not only to a cancelling-out of the stress-induced effect, but also to a prolongation of the anagen of the hair follicle in conditions in which stress is absent.

The NK1-receptor antagonist substance thus also acts as a generic catagen inhibitor.

The experimental evidence described above thus shows that the treatment of the hair follicle with an NK1-receptor antagonist substance can prolong the anagen and substantially limit the effects of stress-induced catagen.

These observations suggest that the results achieved may also be applicable to human beings, resulting in a treatment for human beings against hair loss.

In particular, the treatment can be expected to be particularly effective for the prevention of pathological conditions characterized by premature catagen.

Non-limiting examples of these pathological conditions are androgenetic alopecia, areated alopecia, chronic telogenetic effluvium, and effluvium induced by chemotherapy, by stress, by drugs, by systemic conditions, by endocrine dysfunctions, by zinc or iron deficiency, or by nutritional disorders, but potential benefits of the treatment are also suggested in all other forms of alopecia and effluvium.

Treatment will preferably be topical, although the possibility of systemic treatment is not excluded.

The treatment will preferably be of a preventive nature so that the NK1-receptor antagonist substance will bind to the hair follicle when it is in the anagen (in this connection, it is pointed out that the percentage of hair follicles of the scalp which are in the anagen is normally between 80% and 90%).

However, in addition to a preventive function, treatment with NK1-receptor antagonist substances may be used as a palliative treatment so as to improve the pathological condition.

Any pathological process which can prematurely stimulate catagen in the hair follicles (such as, for example, in hormonal imbalances, in pharmacological treatments, in inflammatory states, in the release of hormones, neuropeptides, or neurotransmitters induced by stress of various kinds, in dietary problems, in various causes of deficiency, etc.) can effectively be opposed by treatment with NK1-receptor antagonistic substances (in their function as catagen inhibitors).

The NK1-receptor antagonist substances usable in connection with the present invention may be those commonly used in other medical fields. Examples of these substances are listed, for example, in United States patents No. 6093748, already mentioned, and No. 5958432.

It is, however, preferable to use, as antagonist substances, compounds constituted by peptides with a number of amino-acids of between 10 and 15. These known compounds are much more specific in their NK1-receptor antagonizing effect and at the same time, have a low degree of toxicity.

It is thus possible to use smaller quantities of antagonist substance, at the same time producing fewer undesired effects.

A further advantageous effect of treatment of the hair follicle with an NK1-receptor antagonist substance is the reduction in inflammatory effects induced in the perifollicular region by stress.

The results discussed above may also advantageously be combined with the already-known observations relating to the stimulating effect of substance P on the hair follicle during the telogen [Paus, R., Heinzelmann, T., Schultz, K.D., Furkert, J., Fechner, K., Czametzki, B.M. (1994) "Hair growth induction by substance P2 *Lab. Invest.* 71, 134-140].

An overall assessment of these results in fact shows a twofold effect of substance P on the hair follicle, depending on the condition of the follicle. Substance P in fact acts as a growth stimulator if applied to hair follicles in the telogen, whereas it acts as a growth inhibitor if applied to follicles in the anagen.

This assessment suggests the possibility of a combined treatment of hair follicles in the telogen, comprising a first follicle-stimulation stage by means of an NK1-receptor agonist substance (for example, substance P itself), directed towards inducing therein a transition to the anagen, and a second stage of maintenance of the anagen by means of an NK1-receptor antagonist substance.

This combined treatment may be used particularly effectively for the curative treatment of the conditions indicated above.

The present invention this achieves the objects proposed, at the same time offering many other advantages, amongst which is the ability to produce a medicament for topical use, also including a substance with a high degree of affinity for the NK1 receptor and having low toxicity.

## Claims

1. Use of an NK 1-receptor antagonist substance for the production of a topical medicament for the treatment of hair loss induced by stress, chemotherapy and/or pharmacological treatments.

2. Use according to claim 1 for the topical treatment of the hair follicle and the contemporaneous localized anti-inflammatory treatment of the perifollicular region.

3. Use of a combination of
i. an NK1-receptor antagonist substance and
ii. an NK1-receptor agonist
for the production of a medicament for the treatment of hair loss induced by stress, chemotherapy and/or pharmacological treatments.

4. Use according to claim 3 for the production of a medicament for the topical treatment of the hair follicle and for the contemporaneous localized anti-inflammatory treatment of the perifollicular region.

5. Use according to any of the claims 1 to 4, **characterised in that** said medicament is to be administered in the anagen of the hair follicle.

6. Use according to any of the claims 1 to 5, **characterised in that** said medicament is to be administered for preventive or palliative treatment of the hair follicle.

7. Use according to any of the claims 1 to 6, **characterised in that** the hair loss is caused by premature catagen of the hair follicle.

8. Use according to the claims 3 or 4, **characterised in that** the treatment with the NK1-receptor agonist substance precedes the treatment with the NK1-receptor antagonist substance.

9. Use according to the claims 3 or 4, **characterised in that** the NK1-receptor agonist is substance P.

10. Use according to the claims 3 and 4, **characterised in that** the NK1-receptor antagonist and the NK1-receptor agonist both are to be, administered topically.

## Patentansprüche

1. Verwendung einer NK-1-Rezeptor-Antagonist-Substanz zur Herstellung eines topisch anzuwendenden Medikaments zur Behandlung von Haarausfall, welcher durch Stress, Chemotherapie und/oder pharmakologische Behandlung ausgelöst wurde.

2. Verwendung nach Anspruch 1 für die topische Behandlung von Haarfollikeln und gleichzeitig erfolgenden anti-inflammatorischen Behandlung der Perifollikularregion.

3. Verwendung einer Mischung aus
(i) einer NK-1 Rezeptor-Antagonist-Substanz und
(ii) einem NK1-Rezeptor Agonisten
zur Herstellung eines topisch anzuwendenden Medikaments zur Behandlung von Haarausfall, welcher durch Stress, Chemotherapie und/oder pharmakologische Behandlung ausgelöst wurde.

4. Verwendung nach Anspruch 3 für die Herstellung eines Medikamentes für die topische Behandlung von Haarfollikeln und die gleichzeitig erfolgende anti-inflammatorische Behandlung der Perifollikularregion.

5. Verwendung nach einem der Ansprüche 1 bis 4, **dadurch gekennzeichnet, dass** das Medikament im Anagen der Haarfollikel angewendet wird.

6. Verwendung nach einem der Ansprüche 1 bis 5, **dadurch gekennzeichnet, dass** das Medikament für die vorbeugende oder palliative Behandlung der Haarfollikel angewendet wird.

7. Verwendung nach einem der Ansprüche 1 bis 6, **dadurch gekennzeichnet, dass** der Haarausfall durch premature Catagen der Haarfollikel ausgelöst wird.

8. Verwendung nach den Ansprüchen 3 oder 4, **dadurch gekennzeichnet, dass** die Behandlung mit der NK1-Rezeptor-Agonist-Substanz der Behandlung mit der NK1-Rezeptor-Antagonist-Substanz vorausgeht.

9. Verwendung nach den Ansprüchen 3 oder 4, **dadurch gekennzeichnet, dass** der NK1-Rezeptor-Agonist Substanz P ist.

10. Verwendung nach den Ansprüchen 3 oder 4, **dadurch gekennzeichnet, dass** NK1-Rezeptor-Antagonist und NK1-Rezeptor-Agonist beide topisch angewendet werden.

## Revendications

1. Utilisation d'une substance récepteur antagoniste de NK-1 pour la préparation d'un médicament à appliquer superficiellement au traitement de la perte de cheveux qui a été déclenchée par un stress, une chimiothérapie et/ou un traitement pharmacologique.

2. Utilisation selon la revendication 1 pour le traitement superficiel de follicules de cheveux et le traitement anti-inflammatoire simultané de la région périfolliculaire.

3. Utilisation d'un mélange
(a) d'une substance récepteur antagoniste de NK-1 et
(b) d'une substance récepteur agoniste de NK-1
pour la préparation d'un médicament à appliquer superficiellement au traitement de la perte de cheveux qui a été déclenchée par un stress, une chimiothérapie et/ou un traitement pharmacologique.

4. Utilisation selon la revendication 3 pour le traitement superficiel de follicules de cheveux et le traitement anti-inflammatoire simultané de la région périfolliculaire.

5. Utilisation selon les revendications 1 à 4, **caractérisée en ce que** le médicament est appliqué dans la phase anagène de follicules de cheveux.

6. Utilisation selon les revendications 1 à 5, **caractérisée en ce que** le médicament est appliqué pour le traitement préventif ou palliatif de follicules de cheveux.

7. Utilisation selon les revendications 1 à 6, **caractérisée en ce que** la perte de cheveux est decléchée par la catagène prématurée de follicules de cheveux.

8. Utilisation selon les revendications 3 ou 4, **caractérisée en ce que** le traitement avec la substance récepteur agoniste de NK-1 est effectué avant le traitement avec la substance récepteur antagoniste de NK-1.

9. Utilisation selon les revendications 3 ou 4, **caractérisée en ce que** la substance récepteur agoniste de NK-1 est la substance P.

10. Utilisation selon les revendications 3 ou 4, **caractérisée en ce que** les deux, la substance récepteur antagoniste de NK-1 et la substance récepteur agoniste de NK-1, on les applique superficiellement.
